# EUROPÄISCHE PATENTSCHRIFT

(11) **EP 1 127 189 B1**
(45) Veröffentlichungstag und Bekanntmachung des Hinweises auf die Patenterteilung: **26.03.2003**
(21) Anmeldenummer: 99963191.4
(22) Anmeldetag: 03.11.1999
(51) Int. Cl.: D21G 9/00, G01N 21/35, G01N 21/89, G01N 33/34

(54) **VERFAHREN UND VORRICHTUNG ZUR MESSUNG DER QUALITÄTSEIGENSCHAFTEN VON PAPIER UND/ODER PAPPE AN LAUFENDEN MATERIALBAHNEN**
METHOD AND DEVICE FOR MEASURING THE QUALITY OF PAPER AND/OR CARDBOARD ON MOVING WEBS
PROCEDE ET DISPOSITIF POUR MESURER LA QUALITE DE PAPIER ET/OU DE CARTON SUR DES BANDES DE MATERIAU EN MOUVEMENT

(30) Priorität: 04.11.1998 DE 19850825
(43) Veröffentlichungstag der Anmeldung: 29.08.2001
(73) Patentinhaber: SIEMENS AKTIENGESELLSCHAFT, 80333 München (DE)
(72) Erfinder: HARTENSTEIN, Hermann, D-91077 Neunkirchen (DE); LAMPE, Uwe, D-21614 Buxtehude (DE); ROTH, Christoph, Shinagawa-ku, Tokyo 141-8641 (JP)
(86) Internationale Anmeldenummer: DE9903510
(87) Internationale Veröffentlichungsnummer: WO00026610

(56) Entgegenhaltungen:
- EP-A- 0 745 917
- WO-A-95/31713
- WO-A-97/38305
- WO-A-98/28488
- DE-A- 19 653 532
- DE-A- 19 827 525

## Beschreibung

Die Erfindung bezieht sich auf ein Verfahren zur Messung der Qualitätseigenschaften von Papier und/oder Pappe an laufenden Materialbahnen, wobei optische Methoden berührungslos eingesetzt werden und wobei kontinuierlich optische.Spektren, vorzugsweise im Infrarot-Bereich, gemessen und mit chemometrischen Methoden ausgewertet werden.

In der nichtvorveröffentlichten DE 198 30 323 Al mit älterem Zeitrang ist ein Verfahren und eine zugehörige Vorrichtung zur Ermittlung der Dicke von Papier oder Pappe bei einer laufenden Materialbahn vorbeschrieben, bei denen berührungslos optisch gemessen wird und die Spektren mit chemometrischen Methoden ausgewertet werden.

Von letzterem ausgehend ist es Aufgabe der Erfindung, das Verfahren zur Erfassung der Eigenschaften von Papier oder Pappe weiterzubilden.

Die Aufgabe ist erfindungsgemäß dadurch gelöst, daß ein gestuftes Auswerteverfahren angewandt wird, mit dem einerseits die Grundeigenschaften des Papiers oder der Pappe und andererseits über eine mehrstufige Modellierung weitere Eigenschaften. ermittelt werden. Als Grundeigenschaften werden vorzugsweise das Flächengewicht, die Feuchte und/oder die Dicke des Papiers bzw. der Pappe bestimmt. Vorzugsweise erfolgt eine dreistufige Modellierung, mit der nacheinander Stoffzusammensetzung, Mahlgrad und weitere Einzelgrößen bestimmt werden.

Besonders vorteilhaft ist bei der Erfindung, daß durch eine geeignete Modellierung online die Auswertung nichttraversierend gemessener Größen erfolgen kann, was mit der älteren DE 198 30 323 A1 allein für die Bestimmung der Papierdicke vorgeschlagen wurde. Nunmehr wird aber nicht nur die Papierdicke bestimmt, sondern es können darüber hinaus alle weiteren interessierenden Qualitätseigenschaften des Papiers oder der Pappe ermittelt werden. Dies ist in geeigneter Weise durch die vorgeschlagene mehrstufige Modellierung möglich.

Weitere Einzelheiten und Vorteile der Erfindung ergeben sich aus der nachfolgenden Figurenbeschreibung von Ausführungsbeispielen anhand der Zeichnung in Verbindung mit weiteren Unteransprüchen.

### Es zeigen

- Figur 1: ein kontinuierliches Spektrum für Papier,
- Figur 2: die Vorgehensweise bei der Auswertung mit einer stufenweisen Modellierung.
- Figur 3: Vergleich von gemessenen und berechneten Werten für den Mahlgrad,
- Figur 4: den Mahlgrad über ein Kollektiv von Proben,
- Figur 5: den Vergleich von berechneten und gemessenen CMT-Werten für Papier,
- Figur 6: den Vergleich von berechneten und gemessenen Werten für den Lichtstreukoeffizient, und
- Figur 7: den Lichtstreukoeffizient über ein Kollektiv von Meßproben.

Gleiche bzw. gleichwirkende Teile haben in den Figuren gleiche Bezugszeichen. Insbesondere die Diagramme werden zur Verdeutlichung des erfindungsgemäßen Vorgehens teilweise gemeinsam beschrieben.

Die Infrarotspektroskopie im Bereich des NIR (Nahes Infrarot) und des MIR (Mittleres Infrarot) stellt eine seit langem bekannte Methode dar, chemische Verbindungen qualitativ anhand ihrer spezifischen Absorption zu identifizieren und anhand der Absorptionsstärke quantitative Aussagen zu machen. Speziell Papier ist ein Stoffgemisch aus dem organischen Grundmaterial - wie gebleichte und ungebleichte Zellstoffe und Holzstoffe -, anorganischen Bestandteilen - wie Füllstoffe und der sog. Strich auf dem fertigen Papier - sowie aus weiteren organischen Hilfsstoffen - wie Leimungsmitteln, naßfesten Mitteln od. dgl.. Es hat sich gezeigt, daß es möglich ist, mit Hilfe der IR-Spektroskopie im Bereich des NIR oder des MIR durch Erfassung und Auswertung von kontinuierlichen Spektren die vorstehend genannten Bestandteile zu identifizieren und quantitativ zu bestimmen.

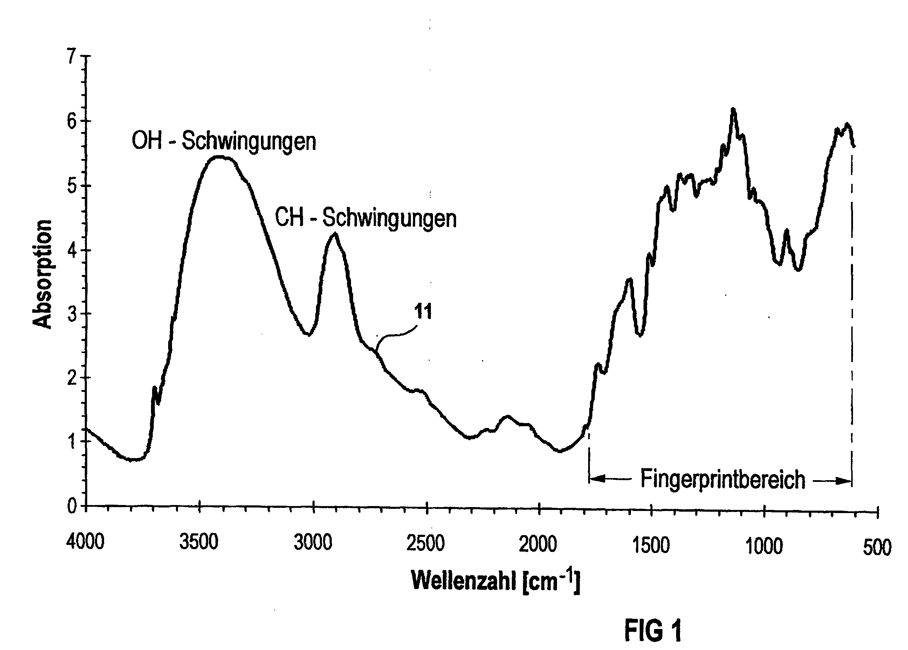

In der Figur 1 ist ein diesbezügliches Spektrum 11 für Papier dargestellt. Aufgetragen ist die Absorption über der Wellenzahl. Wesentlich für die weitere Auswertung ist ein Spektralbereich bei Wellenzahlen zwischen etwa 3500 cm⁻¹ und etwa 3000 cm⁻¹, bei dem signifikante Strukturen von den OH-Schwingungen und den CH-Schwingungen bewirkt werden, sowie der Bereich ab 1800 cm⁻¹, der als sogenannter Fingerprintbereich bezeichnet wird und Aussagen über Qualitätseigenschaften von insbesondere Papier zuläßt.

Relevante Qualitätseigenschaften von Papier, die im wesentlichen auch durch die Dicke des Papiers bestimmt werden, sind im einzelnen beispielsweise der Mahlgrad, der Luftdurchgangswiderstand und die mechanischen Festigkeiten und die optischen Eigenschaften.

Bezüglich des Mahlgrades wird davon ausgegangen, daß durch die Mahlung die Zellstoff- und Holzfasern als Ausgangsstoff für das Papier mechanisch verändert werden, insbesondere verkürzt oder defibriliert. Die Veränderung der Teilchengröße geht unmittelbar in die optischen Eigenschaften, beispielsweise die Lichtstreuung, ein, was durch das Spektrum wiedergegeben wird. Daneben sind auch Änderungen in der chemischen Struktur der Fasern festzustellen. Insbesondere ist der Anteil an Hemizellulosen an der Faseroberfläche sowie die Stärke und die Dichte der inter- und intramolekularen Wasserstoffbrückenverbindungen der Zellulose durch die Mahlung verändert.

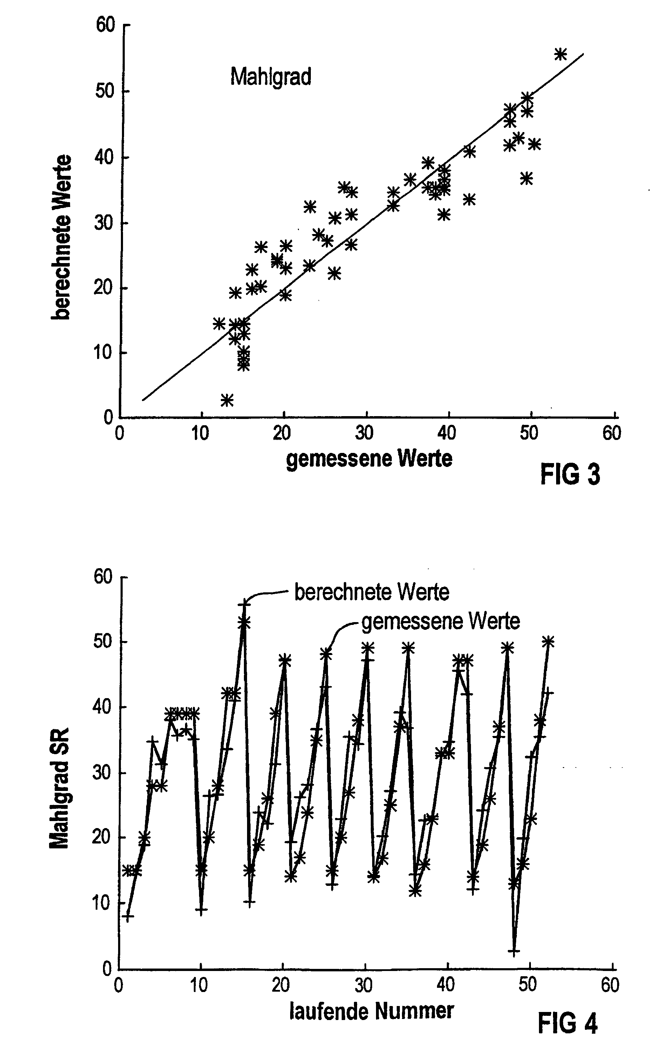

Solche Veränderungen der chemischen Strukturen sind im Infrarotspektrum sichtbar. Insbesondere ist eine Korrelation zwischen den im Spektrum beobachteten Änderungen und dem Mahlgrad festzustellen, was in den Figuren 3 und 4 wiedergegeben ist. Im einzelnen sind in Figur 3 die berechneten Werte des Mahlgrades SR in Abhängigkeit von den gemessenen Werten dargestellt, wobei sich eine hinreichend gute Korrelation zeigt. In Figur 4 ist der Mahlgrad anhand eines Kollektivs von Proben mit gemessenen und berechneten Werten dargestellt. In diesen Figuren beinhalten die Ergebnisse die Modellierung des Mahlgrades auf der Basis der Infrarotspektren für Papier aus gebleichtem Nadelholz bzw. Sulfatzellstoff.

Der Luftdurchgangswiderstand, d.h. die Porosität des Papiers wird im wesentlichen von der Fasergröße und der Dichte der Faserpackung beeinflußt. Durch Untersuchungen wird bestätigt, daß eine Korrelation zu den Strukturen von Infrarotspektren besteht.

Die mechanischen Festigkeiten von Papier korrelieren schließlich analog wie der Mahlgrad mit der Fasergröße, Faserform und der Faserstärke, d.h. insbesondere der Bindungsstärke der Fasern, der Bindungsstärke zwischen den Fasern und zusätzlich der Faserorientierung, welche auch als Formation bezeichnet wird. Die Bindungsstärke innerhalb der und zwischen den Fasern wird hauptsächlich durch die Stärke und Dichte der Wasserstoffbrücken zwischen den Zellulosemolekülen bestimmt. In diesem Zusammenhang wird im einzelnen auf die veröffentlichte WO 97/38305 A1 verwiesen, gemäß der das Vorliegen von an der Faseroberfläche miteinander verbundenen Hydroxylund/oder Carboxylgruppen optisch erfaßt wird.

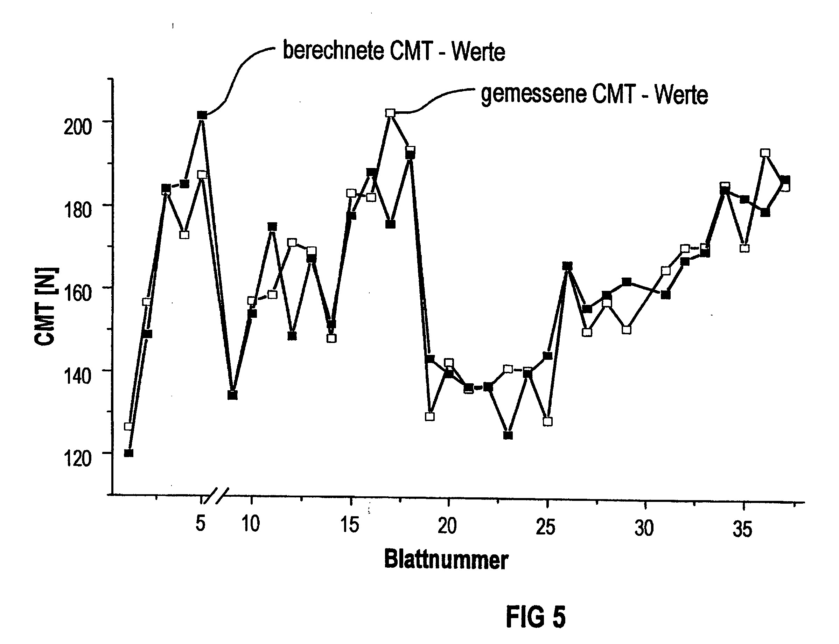

Die vorstehend genannten Faktoren haben Einfluß auf die Spektren von Papieren im NIR(Nahes Infrarot)- oder MIR(Mittleres Infrarot)-Bereich, so daß von einer Korrelation zwischen den Spektren und den mechanischen Festigkeiten ausgegangen werden kann. Diese Voraussagen werden anhand der Figur 5 bestätigt, die die Korrelation der Modellierung des sogenannten CMT-Wertes mit gemessenen Eigenschaften wiedergibt.

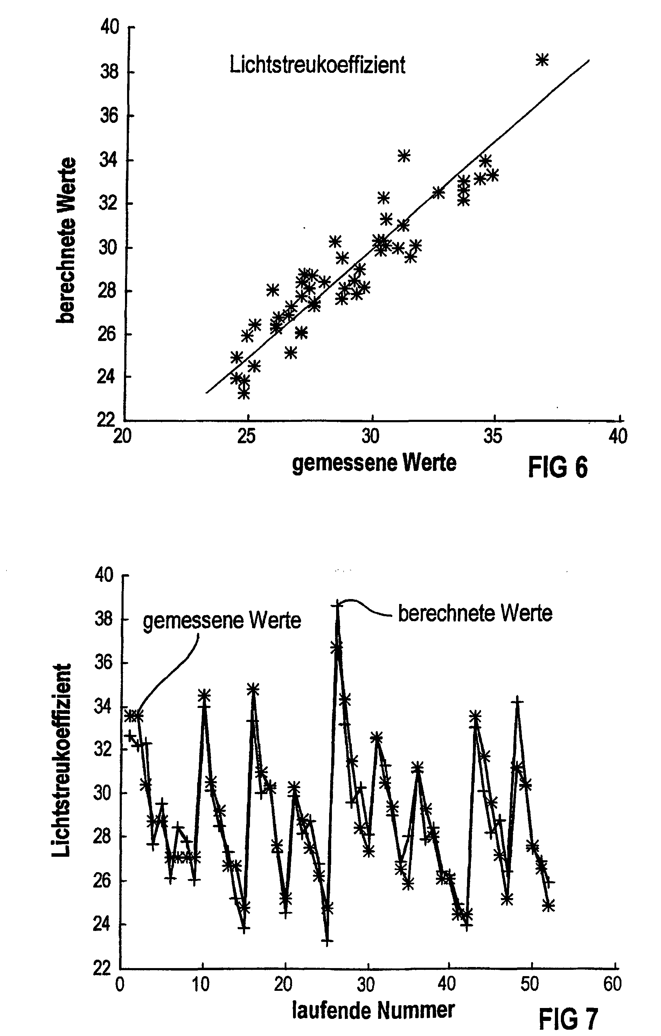

Schließlich sind noch die optischen Eigenschaften von Bedeutung, die unmittelbar durch Messung mit sichtbarem Licht ermittelt werden können. Dabei handelt es sich um Lichtstreueffekte, die auch durch Messung mit IR-Spektren zu bestimmen sind, wie auch um Stoffeigenschaften, die unmittelbar durch die Stoffzusammensetzung bestimmbar sind. Die Figuren 6 und 7 zeigen die Ergebnisse der Modellierung speziell für den Lichtstreukoeffizienten für Papier aus gebleichtem Nadelholzsulfatzellstoff (BSK).

Die Messung der Spektren kann in einfacher Weise insbesondere mit einem Sensorarray entsprechend der älteren deutschen Patentanmeldung 198 30 323.8 vorgenommen werden, wobei das Array in einem die laufende Papierbahn überspannenden Meßrahmen integriert ist. Zur Erhöhung der Zahl der Meßpunkte kann eine Anlage vorgesehen sein, mit dem der gesamte Meßrahmen quer zur Papierbahn bewegt wird, entsprechend der älteren deutschen Patentanmeldung 197 29 005.1, wobei die Messung mit der Rahmenbewegung synchronisiert wird. Wie bereits erwähnt, erfolgt die Messung im Bereich des Infraroten, wobei kontinuierliche Spektren mit Wellenlängen von 1 bis 2,5 µm (NIR) und/oder 2,5 bis 20 µm (MIR) erfaßt werden. Die Messung erfolgt in Abhängigkeit vom Flächengewicht in Transmission oder Diffusion bzw. direkter Reflexion.

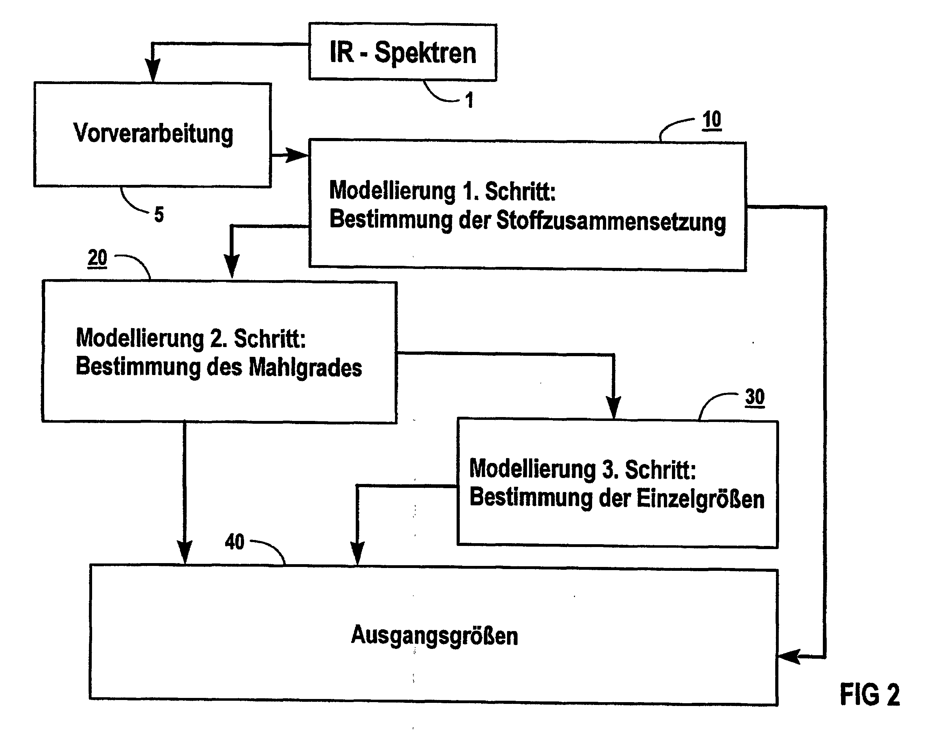

Die Auswertung der so erhaltenen Spektren erfolgt nach mathematischer Vorverarbeitung mit Hilfe von chemometrischen Methoden, wie vorzugsweise die bekannten Methoden des Partial Least Square (PLS) oder Partial Component Analysis (PCA = Hauptkomponentenanalyse) und der multivariablen Datenanalyse bzw. Einsatz von neuronalen Netzen. Es wird nunmehr ein mehrstufiges Verfahren zur Analyse eingesetzt, wie es im einzelnen anhand Figur 2 dargestellt wird.

In Figur 2 ist ein Erfassungsblock für die gemessenen IR-Spektren mit 1 bezeichnet. In einer Einheit 5 wird entsprechend dem Stand der Technik eine Vorverarbeitung vorgenommen, der sich nunmehr einzelne Modellierungseinheiten anschließen:

In einer ersten Modellierungseinheit 10, in die als Input die vorverarbeiteten Spektren zur weiteren Auswertung gegeben werden, wird in einem ersten Schritt der Modellierung die Stoffzusammensetzung bestimmt. Als Ausgang wird beispielsweise der Gehalt an Faserstoffen, an Füllstoffen und an Hilfsstoffen ausgegeben. Auch der sog. Strich als Papiereigenschaft kann bestimmt und ausgegeben werden.

In einer zweiten Modellierungseinheit 20, in die als Input die Ergebnisse der ersten Modellierungseinheit 10, d.h. die bearbeiteten Spektren und insbesondere der Gehalt an Faserstoffen, eingegeben werden, wird im zweiten Schritt eine weitere Modellierung aus dem Gehalt der Faserstoffe der Mahlgrad bestimmt. Speziell der Mahlgrad wird als Ergebnis der zweiten Modellierungseinheit 20 angegeben und in einer dritten Modellierungseinheit 30 weiter ausgewertet.

Unter Eingabe der bearbeiteten Spektren, dem Gehalt an Faserstoffen, dem Mahlgrad, dem Gehalt an Füllstoffen und den weiteren Hilfsstoffen werden anschließend in der Einheit 40 die Einzelgrößen ausgewertet. Dabei wird gleichermaßen der Mahlgrad als Ergebnis des zweiten Modellierungsschrittes berücksichtigt.

Als Modelloutput in der Einheit 40 ergeben sich jeweils getrennte Modelle für die Grundgrößen, für die Zusammensetzung, für die mechanische Festigkeit und für die optischen Eigenschaften. Als Grundgrößen werden das Flächengewicht, die Feuchte, die Dicke und die Asche des Papiers, als Größen für die Zusammensetzung dagegen die Füllstoffe, der Strich und die Hilfsstoffe angegeben. Als Größen für die mechanischen Eigenschaften ergeben sich die Reißfestigkeit, die Berstfestigkeit und die Weiterreißarbeit als für die praxiswesentliche Qualitätsparameter. Als Größen für die optischen Eigenschaften ergeben sich schließlich die Lichtabsorption, Opazität und die Lichtstreuung. Mit der Gesamtheit dieser Größen können Papier und/oder Pappe in ihren Qualitätseigenschaften umfassend beschrieben werden.

In der Figur 2 umfaßt die Einheit 1 zur Vorverarbeitung der Spektren die Bildung von Mittelwerten, das Glätten der Spektren und die Bildung von Ableitungen. Dabei können stark gestörte Teile der Spektren für die Weiterverarbeitung eliminiert werden. In den anschließenden Einheiten 10, 20 und 30 werden für jede der zu bestimmenden Größen eigene Modelle erstellt, wobei zur Berechnung der einzelnen Meßgrößen im Einzelfall auch unterschiedliche Spektralbereiche und Vorverarbeitungen zweckmäßig sind. Wie erwähnt, werden für die Modellierung chemometrische Methoden, wie insbesondere PCA und PLS, eine multivariable Datenanalyse und/oder neuronale Netze eingesetzt. Wie im einzelnen beschrieben, setzt sich die Modellierung aus drei Schritten zusammen, die nachfolgend mit ihren Eigenschaften nochmals zusammengefaßt sind:

Der erste Schritt der Modellierung gemäß Einheit 10 umfaßt die Bestimmung der qualitativen und quantitativen stofflichen Zusammensetzung des Papiers. Er erfolgt eine Klassifizierung nach Faserstoffen und Bestimmung deren Anteils, und zwar im einzelnen zu
- BSK = bleached softwood Kraft (gebleichter Nadelholz-Sulfatzellstoff)
- BHK = bleached hardwood Kraft (gebleichter Laubholz-Sulfatzellstoff)
- BSS = bleached hardwood Sulfit (gebleichter Laubholz Sulfitzellstoff)
- UBSK = unbleached softwood Kraft (ungebleichter Nadelholz-Sulfatzellstoff)
- TMP = thermo mechanical Pulp (Refinerstoff)
- CTMP = chemo thermo mechanical Pulp (Refinerstoff mit chemischer Umsetzung)
- GW = ground Wood (Holzschliff).

Diese Größen können als Auswahlkriterium für das Modell des nächsten Berechnungsschrittes dienen, und zwar der Bestimmung der Füllstoffe und des sog. Strichs, d.h. der Beschichtung des Papiers. Dies sind im einzelnen
- Calciumcarbonat
- Kaolin
- Titandioxid
- Schwerspat

Die Modellgrößen können unmittelbar eine zu bestimmende Meßgröße bzw. ein Qualitätsparameter sein und zur Regelung des Füllstoffeintrages bzw. des Streichprozesses dienen oder als Eingangsparameter für die nachfolgenden Modelle benutzt werden. Weiterhin erfolgt eine Bestimmung der Hilfsstoffe, wie beispielsweise der Leimungsmittel oder Naßfeststoffe.

Mit den im ersten Schritt der Modellierung ausgewählten Modellen wird der Mahlgrad bestimmt. In Abhängigkeit von den in der speziellen Anwendung bei beispielsweise produzierten Papieren erfaßten Stoffzusammensetzung müssen mehrere Modelle für die Mahlgradberechnung aufgestellt werden. Der Ausgang dieses Modelles kann auch unmittelbar eine zu bestimmende Meßgröße sein und wird z.B. für die Regelung der vor der Papiermaschine eingesetzten Refinern benutzt. Anhand der Stoffzusammensetzung und des danach bestimmten Mahlgrades wird ein geeignetes Modell für die Berechnung der weiteren Papiereigenschaften bestimmt.

Abhängig von der Stoffart gemäß Einheit 10 und dem Mahlgrad gemäß Einheit 20 werden im dritten Schritt in der Einheit 30 mit einzelnen Teilmodellen die Grundeigenschaften
- Flächengewicht
- Feuchte
- Dicke
- Luftdurchgangswiderstand
des Papiers bestimmt. Weiterhin lassen sich mit geeigneten Teilmodellen die mechanischen Festigkeiten wie
- Reißlänge
- Berstfestigkeit
- Weiterreißarbeit
- E-Modul
- Bruchdehnung
sowie die optischen Eigenschaften wie
- Lichtstreukoeffizient
- Lichtabsorptionskoeffizienten
- Opazität
- Reflexionsfaktor
bestimmen. Die Zahl der einzusetzenden Modelle richtet sich dabei nach der Zahl der zu messenden Größen, der Stoffvielfalt und den Mahlgraden.

Die Auswahl des jeweils für die Stoffzusammensetzung und des Mahlgrades gültigen Modelles kann auf der Basis der Auswertung der Infrarotspektren automatisch erfolgen. Zur Unterstützung kann jedoch eine Sollwertvorgabe von der Papierrezeptur und dem Mahlungsgrad vorgesehen werden. Die Ausgabe aller Eigenschaften erfolgt in der Einheit 40.

Die Auswertung der kontinuierlichen Spektren entsprechend der oben beschriebenen Vorgehensweise erfolgt in einem Rechner, in dessen Speicher die oben im einzelnen beschriebenen Modelle bzw. Teilmodelle abgespeichert sind. Durch die software-mäßige Auswertung ergibt sich eine optimale Anpassung bei der Modellierung. Insbesondere werden für die einzelnen Teilmodelle lineare Modellverfahren eingesetzt.

Die Modellierung kann dabei viel zielgerichteter und damit genauer erfolgen als dies mit einem globalen Modell einer Meßgröße über alle Stoffzusammensetzungen und Mahlgraden erreichbar wäre. Dabei kann analytisches Wissen einbezogen werden. Die stufenweise Auswertung ermöglicht und erleichtert die Einbeziehung von analytisch bekannten Zusammenhängen.

Anhand der Figuren 3 bis 7 kann die Wirksamkeit der neuen Vorgehensweise belegt werden. So ergeben insbesondere die Gegenüberstellungen von gemessenen und berechneten Werten für den Mahlgrad SR anhand Figur 3 und den Lichtstreukoeffizient anhand Figur 6 eindeutige Korrelationen. Gleiches gilt für die Darstellung letzterer Größen anhand Figur 4 und Figur 7 für laufende Proben eines Kollektivs. Speziell in Figur 5 ist der sogenannte CMT(chemical mechanical temperature)-Wert als praxisrelevante Kenngröße für die mechanischen Eigenschaften von zellstoffbasierten Materialien dargestellt. Auch hier ergibt sich eine gute Übereinstimmung.

Das Verfahren wurde im einzelnen für Papier als Materialbahn beschrieben. Wie bereits angedeutet, gilt Entsprechendes aber auch für Pappe als Zellstoff- und Holzstoff enthaltendes Material.

## Patentansprüche

1. Verfahren zur Messung der Qualitätseigenschaften von Papier und/oder Pappe an laufenden Materialbahnen, wobei optische Methoden berührungslos eingesetzt werden, und wobei kontinuierliche optische Spektren, vorzugsweise im Infrarot(IR)-Bereich, gemessen und die Spektren mit chemometrischen Methoden ausgewertet werden,
wobei ein gestuftes Auswerteverfahren angewandt wird, mit dem einerseits die Grundeigenschaften des Papiers bzw. der Pappe und andererseits über eine mehrstufige Modellierung weitere Eigenschaften ermittelt werden.

2. Verfahren nach Anspruch 1, **dadurch gekennzeichnet, daß** als Grundeigenschaften des Papiers bzw. der Pappe Flächengewicht, Feuchte und/oder Dicke bestimmt werden.

3. Verfahren nach Anspruch 1, **dadurch gekennzeichnet, daß** eine dreistufige Modellierung durchgeführt wird, wobei
- im ersten Modellierungsschritt eine Bestimmung der Stoffzusammensetzung erfolgt,
- im zweiten Modellierungsschritt eine Bestimmung des Mahlgrades erfolgt und
- im dritten Modellierungsschritt eine Bestimmung von Einzelgrößen erfolgt.

4. Verfahren nach Anspruch 3, **dadurch gekennzeichnet, daß** bei der Bestimmung der Stoffzusammensetzung der Gehalt an Faserstoffen, Füllstoffen und Hilfsstoffen, wie insbesondere Leimungsmittel, erfaßt wird.

5. Verfahren nach Anspruch 3, **dadurch gekennzeichnet, daß** durch Bestimmung des Mahlgrades die mechanischen Werte des Papiers bzw. der Pappe, wie insbesondere Reißlänge, Berstfestigkeit, Weiterreißfestigkeit, E-Modul, Bruchdehnung, ermittelt werden.

6. Verfahren nach Anspruch 3, **dadurch gekennzeichnet, daß** zur Bestimmung von Einzelgrößen optische Eigenschaften, wie Lichtstreukoeffizient, Lichtabsorptionskoeffizient, Opazität, Reflexionsfaktor, erfaßt werden.

7. Verfahren nach einem der vorhergehenden Ansprüche, **dadurch gekennzeichnet, daß** ein Querprofil der Eigenschaften des Papiers bzw. der Pappe an der laufenden Materialbahn bestimmt wird.

8. Verfahren nach Anspruch 7, **dadurch gekennzeichnet**, die Messung der Daten zur Bestimmung der Eingangsgrößen für die Modellierung ohne Traversierung auf der Materialbahn erfolgt.

9. Verfahren nach Anspruch 8, **dadurch gekennzeichnet, daß** die Messung mit einem Sensorarray erfolgt.

## Claims

1. Method of measuring the quality properties of paper and/or board on moving webs, optical methods without contact being used and continuous optical spectra, preferably in the infra-red (IR) range, being measured and the spectra being evaluated by chemometric methods, in which a staged evaluation method is applied in which, firstly, the basic properties of the paper or board and, secondly, further properties are determined via multi-stage modelling.

2. Method according to Claim 1, **characterized in that** the basic properties of the paper or board which are determined are the grammage, moisture and/or thickness (caliper).

3. Method according to Claim 1, **characterized in that** three-stage modelling is carried out,
- in the first modelling step, the material composition being determined,
- in the second modelling step, the freeness being determined and
- in the third modelling step, individual variables being determined.

4. Method according to Claim 3, **characterized in that** during the determination of the material composition, the content of fibrous materials, cellulose and auxiliary materials, such as in particular sizing agents, is registered.

5. Method according to Claim 3, **characterized in that** by determining the freeness, the mechanical values of the paper or board, such as in particular tearing lengths, bursting strength, tear propagation resistance, modulus of elasticity, extension at break, are determined.

6. Method according to Claim 3, **characterized in that** in order to determine individual variables, optical properties such as light scattering coefficient, light absorption coefficient, opacity, reflectance, are determined.

7. Method according to one of the preceding claims, **characterized in that** a cross-machine profile of the properties of the paper or board is detected on the moving web.

8. Method according to Claim 7, **characterized in that** the measurement of the data for determining the input variables for the modelling is carried out on the material web without traversing.

9. Method according to Claim 8, **characterized in that** the measurement is carried out with a sensor array.

## Revendications

1. Procédé pour la mesure des propriétés de qualité de papier et/ou de carton sur des bandes de matériau en mouvement, dans lequel on utilise des méthodes optiques sans contact, dans lequel on mesure des spectres optiques continus, de préférence dans la plage infrarouge (IR), et on évalue les spectres avec des méthodes chimiométriques et dans lequel on applique un procédé d'évaluation à plusieurs niveaux avec lequel on détermine d'une part les propriétés fondamentales du papier ou du carton et d'autre part d'autres propriétés par l'intermédiaire d'une modélisation à plusieurs niveaux.

2. Procédé selon la revendication 1, **caractérisé par le fait qu'**on détermine comme propriétés fondamentales du papier ou du carton le grammage, l'humidité et/ou l'épaisseur.

3. Procédé selon la revendication 1, **caractérisé par le fait qu'**on effectue une modélisation à trois niveaux dans laquelle
- on effectue une détermination de la composition du papier ou du carton dans la première étape de modélisation,
- on effectue une détermination de l'indice d'égouttage dans la deuxième étape de modélisation et
- on effectue une détermination de grandeurs individuelles dans la troisième étape de modélisation.

4. Procédé selon la revendication 3, **caractérisé par le fait que**, lors de la détermination de la composition du papier ou du carton, on détecte la teneur en matières fibreuses, en matières de charge et en matières auxiliaires, notamment en agents de collage.

5. Procédé selon la revendication 3, **caractérisé par le fait que**, en déterminant l'indice d'égouttage, on détermine les valeurs mécaniques du papier ou du carton, notamment la longueur de déchirure, la résistance à l'éclatement, la résistance à une déchirure amorcée, le coefficient d'élasticité et l'allongement à la rupture.

6. Procédé selon la revendication 3, **caractérisé par le fait que**, pour la détermination de grandeurs individuelles, on détecte des propriétés optiques, par exemple le coefficient de dispersion de la lumière, le coefficient d'absorption de la lumière, l'opacité, le facteur de réflexion.

7. Procédé selon l'une des revendications précédentes, **caractérisé par le fait qu'**on détermine un profil transversal des propriétés du papier ou du carton sur la bande de matériau en mouvement.

8. Procédé selon la revendication 7, **caractérisé par le fait que** la mesure des données pour la détermination des grandeurs d'entrée pour la modélisation s'effectue sans traversée de la bande de matériau.

9. Procédé selon la revendication 8, **caractérisé par le fait que** la mesure s'effectue avec un réseau de capteurs.
